(19) Europäisches Patentamt / European Patent Office / Office européen des brevets

(11) **EP 3 462 175 A1**

(12) **EUROPEAN PATENT APPLICATION**

(43) Date of publication:
03.04.2019 Bulletin 2019/14

(51) Int Cl.:
**G01N 33/543** (2006.01)    **C07K 16/18** (2006.01)
**G01N 21/64** (2006.01)

(21) Application number: **18195594.9**

(22) Date of filing: **20.09.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.09.2017   JP 2017190652**

(71) Applicant: **KONICA MINOLTA, INC.**
**Tokyo**
**100-7015 (JP)**

(72) Inventors:
• **Inui, Chie**
  **Tokyo, 100-7015 (JP)**
• **Maezawa, Akihiro**
  **Tokyo, 100-7015 (JP)**

(74) Representative: **Gille Hrabal**
**Brucknerstrasse 20**
**40593 Düsseldorf (DE)**

(54) **ANTIBODY DISPERSION FOR MEASUREMENT, PRODUCTION METHOD THEREOF, KIT FOR PREPARING ANTIBODY DISPERSION FOR MEASUREMENT, AND METHOD FOR MEASURING BIOLOGICAL SUBSTANCE**

(57)    An object of the invention is to provide an antibody dispersion for measurement, in which an antibody is favorably dispersed when used for a measurement, a production method thereof, a kit for preparing an antibody dispersion for measurement, and a measuring method of a biological substance.

An antibody dispersion for measurement of the invention includes a monovalent metal salt and a divalent metal salt, in which the concentration of the monovalent metal salt is from 50 to 500 mM and the concentration of the divalent metal salt is from 1.0 to 50 mM

**EP 3 462 175 A1**

**Description**

Technological Field

The present invention relates to an antibody dispersion for measurement, a production method thereof, a kit for preparing antibody dispersion for measurement, and a method for measuring biological substance.

Background

Detection and quantification of a tumor marker, a specific protein, another antigen contained in human or animal blood, urine, or another biological sample are widely practiced in diagnosis in today's medical field, or researches in the field of biology and biochemistry. Meanwhile, as a method for detecting specifically a trace amount of a target biological substance (antigen), an immunoassay method is used. As an immunoassay method, a sandwich assay using an antibody for capturing an antigen and an antibody for labeling, a radioimmunoassay using a label of a radioactive substance, and the like are widely practiced.

A sandwich assay, which is one of immunoassay methods, can detect a trace amount of a target biological substance (antigen), and various detection methods have been known. As one of such detection methods, there is, for example, surface plasmon field-enhanced fluorescence spectroscopy (SPFS) utilizing a surface plasmon resonance phenomenon, by which an analyte may be detected with high accuracy.

In the surface plasmon field-enhanced fluorescence spectroscopy (SPFS), the surface of a metal thin film is irradiated with excitation light such as laser beam from a light source under conditions that attenuated total reflectance (ATR) occurs, so that surface plasmon light (compression wave) is generated on the surface of a metal thin film to increase the amount of photons included in the excitation light emitted from the light source several tens to several hundreds times achieving an electric field enhancing effect of plasmon surface plasmon light.

The surface plasmon field-enhanced fluorescence spectroscopy (SPFS) is capable of exciting efficiently a fluorescent substance bound (labeled) to an analyte captured near the surface of the metal thin film owing to the electric field enhancing effect, so that by observing this fluorescence, an analyte in an extremely small amount and at an extremely low concentration may be detected.

For quantifying a trace amount of an antigen contained in a biological sample using surface plasmon field-enhanced fluorescence spectroscopy (SPFS), it is necessary to react efficiently and accurately the antigen with a fluorescence-labeled antibody.

A technique to add a surfactant to an antibody dispersion used for labeling an antigen has been known (see, for example, Patent Document 1).

Related Art Document

Patent Document

[Patent Document 1] Japanese Patent Application Laid-Open No. H4-48266

Summary

As a result of intensive investigations by the present inventors, it has been found that the efficiency and reactivity of an antigen-antibody reaction may be enhanced to improve the accuracy of quantification, by controlling the aggregation, or dispersion of a fluorescence-labeled antibody, and the folding structure at the time of measurement. However, it has been also found that, when an antibody dispersion in which the fluorescence-labeled antibody has been stably preserved is used, aggregation of the fluorescence-labeled antibody occurs and the efficiency of the antigen-antibody reaction decreases, and that the quantum yield of fluorescence decreases due to short distance among individual fluorescence-labeled antibodies to decrease the emission intensity and therefore the accuracy of quantification is lowered.

Meanwhile, the present inventors have also studied to favorably disperse the antibody at the time of measurement using a surfactant. However, since the method using a surfactant takes time to disperse the antibody, there has been a drawback in that the method is not usable in a scene such as an emergency case in the medical field, where a quick measurement is essential.

In view of such a present situation, an object of the present invention is to provide an antibody dispersion for measurement in which the antibody is favorably dispersed when used for measurement, a production method thereof, a kit for preparing an antibody dispersion for measurement, and a method for measuring a biological substance.

In other words, the present invention provides an antibody dispersion for measurement, a production method thereof, a kit for preparing an antibody dispersion for measurement, and a method for measuring a biological substance, for example described in the following [1] to [17].

[1] An antibody dispersion for measurement comprising a monovalent metal salt and a divalent metal salt, wherein the concentration of the monovalent metal salt is from 50 to 500 mM and the concentration of the divalent metal salt is from 1.0 to 50 mM

[2] The antibody dispersion for measurement according to [1] above comprising a fluorescence-labeled antibody.

[3] The antibody dispersion for measurement according to [1] or [2] above obtained by adding a divalent metal salt to an antibody dispersion for preservation containing a monovalent metal salt at a concentration of from 10 to 300 mM

[4] The antibody dispersion for measurement according to [1] or [2] above obtained by adding a divalent metal salt and a monovalent metal salt to an antibody dispersion for preservation containing a monovalent metal salt at a concentration of from 10 to 300 mM

[5] The antibody dispersion for measurement according to [3] or [4] above, wherein the sum of the concentration of the monovalent metal salt and the concentration of the divalent metal salt contained in the antibody dispersion for measurement is higher than the sum of the concentration of the monovalent metal salt and the concentration of the divalent metal salt contained in the antibody dispersion for preservation by 0.5 to 250 mM

[6] The antibody dispersion for measurement according to any one of [1] to [5] above containing a fluorescence-labeled antibody, wherein the emission intensity of the antibody dispersion for measurement is from 60 to 99%, when the concentration of the fluorescence-labeled antibody contained in the antibody dispersion for measurement is X [mol/L] and the emission intensity of a dispersion of a bare fluorescent substance to constitute a fluorescence-labeled antibody (at a concentration of X [mol/L]) is regarded as 100%.

[7] The antibody dispersion for measurement according to any one of [1] to [6] above, wherein the divalent metal salt is at least one metal salt selected from a magnesium salt and a calcium salt.

[8] The antibody dispersion for measurement according to any one of [1] to [7] above, wherein the monovalent metal salt is at least one metal salt selected from a sodium salt, a potassium salt, and a lithium salt.

[9] A production method of an antibody dispersion for measurement comprising a monovalent metal salt and a divalent metal salt, wherein the concentration of the monovalent metal salt is from 50 to 500 mM and the concentration of the divalent metal salt is from 1.0 to 50 mM, and
wherein the method comprises a step of adding a divalent metal salt to an antibody dispersion for preservation containing a monovalent metal salt at a concentration of from 10 to 300 mM

[10] The production method of an antibody dispersion for measurement according to [9] above, wherein the antibody dispersion for preservation contains a fluorescence-labeled antibody.

[11] The production method of an antibody dispersion for measurement according to [9] or [10] above, wherein the step of adding a divalent metal salt is a step of adding a divalent metal salt and a monovalent metal salt.

[12] The production method of an antibody dispersion for measurement according to any one of [9] to [11] above comprising a step of adding a monovalent metal salt after the step of adding a divalent metal salt.

[13] The production method of an antibody dispersion for measurement according to any one of [9] to [12] above, wherein the divalent metal salt is at least one metal salt selected from a magnesium salt and a calcium salt.

[14] The production method of an antibody dispersion for measurement according to any one of [9] to [13] above, wherein the monovalent metal salt is at least one metal salt selected from a sodium salt, a potassium salt, and a lithium salt.

[15] A kit for preparing an antibody dispersion for measurement comprising an antibody dispersion for preservation containing a monovalent metal salt at a concentration of from 10 to 300 mM, and a solution containing a divalent metal salt,
wherein an antibody contained in the antibody dispersion for preservation is a fluorescence-labeled antibody.

[16] A measuring method of a biological substance for quantifying a target biological substance comprising the following steps (A) to (D):

(A) a step for obtaining an antibody dispersion for measurement, in which a monovalent metal salt and a divalent metal salt are contained, and the concentration of the monovalent metal salt is from 50 to 500 mM and the concentration of the divalent metal salt is from 1.0 to 50 mM, by adding a divalent metal salt to an antibody dispersion for preservation, in which a fluorescence-labeled antibody and a monovalent metal salt are contained, and the concentration of the monovalent metal salt is from 10 to 300 mM,
(B) a step for supplying a specimen containing a target biological substance onto a sensor chip,
(C) a step for fluorescently labeling the specimen containing a target biological substance by supplying the antibody dispersion for measurement obtained in the step (A) onto the sensor chip having undergone the step (B), and
(D) a step for detecting the fluorescence-labeled specimen containing a target biological substance by performing a fluorescence assay.

[17] The measuring method of a biological substance according to [16] above, wherein the fluorescence assay is carried out by surface plasmon field-enhanced fluorescence spectroscopy (SPFS).

According to the present invention, an antibody dispersion for measurement in which an antibody is favorably dispersed when it is used for a measurement, a production method thereof, a kit for preparing an antibody dispersion for measurement, and a method for measuring a biological substance may be provided.

Detailed Description of Embodiments

Next, the present invention will be described more specifically.

«Antibody dispersion for measurement»

An antibody dispersion for measurement according to the present invention is an antibody dispersion for measurement in which a monovalent metal salt and a divalent metal salt are contained, and the concentration of the monovalent metal salt is from 50 to 500 mM, and the concentration of the divalent metal salt is from 1.0 to 50 mM

In some case, for example, with respect to a biological sample as a specimen, a fluorescence assay method such as surface plasmon field-enhanced fluorescence spectroscopy (SPFS) may be used for detecting a trace amount of an analyte contained in the specimen. And for detecting the analyte by this fluorescence assay method, labeling may be carried out using an antibody in an antibody dispersion. Such an antibody dispersion used for the labeling is denoted herein an antibody dispersion for measurement.

The present inventors have found that a preferable concentration of a metal salt in an antibody dispersion differs between at the time of distribution, storage, *etc.* and at the time of use.

An antibody dispersion to be distributed, stored, *etc.* is denoted herein as an antibody dispersion for preservation, and an antibody dispersion to be used for measurement is denoted as an antibody dispersion for measurement.

Examples of a monovalent metal salt include an alkali metal salt, such as a lithium salt, a sodium salt, and a potassium salt, and at least one metal salt selected from a sodium salt, a potassium salt, and a lithium salt is preferable. A monovalent metal salt is preferably a halide, such as a chloride or a fluoride, a sulfate, or a nitrate. As a monovalent metal salt, sodium chloride, or potassium chloride is more preferable, because they are components contained in blood and the like, which may become a measuring object. Monovalent metal salts may be used singly, or two or more thereof may be used together.

The concentration of a monovalent metal salt in an antibody dispersion for measurement is in the range of 50 to 500 mM from the viewpoints of osmotic pressure and protein aggregation, and preferably in the range of 50 to 300 mM

Examples of a divalent metal salt include a salt of an alkaline earth metal, such as a magnesium salt, and a calcium salt, and an aluminum salt, and at least one metal salt selected from a magnesium salt and a calcium salt is preferable. A divalent metal salt is preferably a halide, such as a chloride or a fluoride, a sulfate, and a nitrate. As a divalent metal salt, magnesium chloride and calcium chloride are more preferable, because the osmotic pressure and protein aggregation may be easily controlled. Divalent metal salts may be used singly, or two or more thereof may be used together.

The concentration of a divalent metal salt in an antibody dispersion for measurement is from the viewpoint of osmotic pressure and protein aggregation in the range of 1.0 to 50 mM, preferably in the range of 3.0 to 40 mM, and more preferably in the range of 8.0 to 35 mM

An antibody dispersion for measurement according to the present invention may be prepared, for example, by adding a divalent metal salt, or a divalent metal salt and a monovalent metal salt to an antibody dispersion for preservation. The addition of a metal salt to an antibody dispersion for preservation may be carried out by directly adding a metal salt, or by adding a solution containing a metal salt.

When an antibody dispersion containing a divalent metal salt in the above range is stored for a long period of time, antibody aggregation or the like may occur and the state of the antibody may change. Therefore, it is preferable to finish an addition operation to a sensor chip, which performs the fluorescence assay to be described later, within 60 min, preferably within 10 min after obtaining an antibody dispersion for measurement. Meanwhile, from the viewpoint of operability, it is preferable to add an antibody dispersion for measurement to a sensor chip, which performs the fluorescence assay to be described later, normally after an elapse of 1 sec or more from obtaining the same.

An antibody contained in an antibody dispersion for measurement according to the present invention is preferably a fluorescence-labeled antibody. The antibody and the fluorescence-labeled antibody will be described in detail in the paragraph on an antibody dispersion for preservation described below.

The concentration of an antibody contained in an antibody dispersion for measurement is preferably in the range of 0.0005 to 1 mg/mL, and more preferably from 0.003 to 0.01 mg/mL. When the concentration of an antibody is too low, the rate of an antigen-antibody reaction slows down and the signal decreases. When the concentration of the antibody is too high, the amount of nonspecific adsorption of an antibody to the substrate increases at the time of measurement to generate a noise. Therefore, the above-mentioned range is preferable.

<Antibody dispersion for preservation>

An antibody dispersion for preservation is required to contain a dispersed antibody to a detection target substance, and may contain further a surfactant or the like. As the dispersion medium, water is usually used. It is preferable that an antibody dispersion for preservation is a buffer solution from the viewpoint of pH stabilization. When it is a buffer solution, its examples may include an acetate buffer, a phosphate buffer, a Tris buffer, a HEPES buffer, a citrate buffer, a citrate-phosphate buffer, and a borate buffer. As the buffer solution, among a phosphate buffer, a Tris buffer, and a HEPES buffer, a phosphate buffered saline (PBS), a Tris buffered saline (TBS), and a HEPES buffered saline, which are almost isotonic with a body fluid, are preferable.

Although there is no particular restriction on an antibody to a detection target substance contained in an antibody dispersion for preservation insofar as it causes an antigen-antibody reaction involving the detection target substance as an antigen, an antibody that can be produced by a general method is preferable. As the antibody, a monoclonal antibody is preferable rather than a polyclonal antibody from the viewpoint of stability (reproducibility) of measurement. Examples of a monoclonal antibody include anti-$\alpha$-fetoprotein (AFP) monoclonal antibody (available from Nippon Medical Clinical Laboratory Research Institute, *etc*.), anti-carcinoembryonic antigen (CEA) monoclonal antibody, anti-CA 19-9 monoclonal antibody, and anti-PSA monoclonal antibody.

The concentration of an antibody to the detection target substance contained in an antibody dispersion for preservation is preferably in the range of 0.001 to 2 mg/mL, and more preferably from the viewpoints of securance of the intensity of a fluorescent signal and suppression of nonspecific adsorption of the antibody to the substrate at the time of measurement in the range of 0.006 to 0.02 mg/mL.

Also, the antibody is preferably a fluorescence-labeled antibody bound to a fluorescent substance.

The fluorescent substance is a generic name of a substance that emits fluorescence when irradiated with certain excitation light, or excited by utilizing an electric field effect, and the fluorescence includes also various kinds of luminescence such as phosphorescence. The type of the fluorescent substance used according to the present invention is not particularly limited, and any of known fluorescent substances may be used.

As the fluorescent substance, a fluorescent substance having a large Stokes shift, which exhibits generally excellent detection efficiency, is preferable.

Examples of such a fluorescent substance include:

Fluorescent substance of fluorescein family (produced by Integrated DNA Technologies, Inc.),
Fluorescent substance of polyhalofluorescein family (produced by Applied Biosystems Japan Ltd.),
Fluorescent substance of hexachlorofluorescein family (produced by Applied Biosystems Japan Ltd.),
Fluorescent substance of coumarin family (produced by Invitrogen),
Fluorescent substance of rhodamine family (produced by GE Healthcare Bio-Science Co., Ltd.),
Fluorescent substance of cyanine family,
Fluorescent substance of indolcarbocyanine family, Fluorescent substance of oxazine family,
Fluorescent substance of thiazine family, Fluorescent substance of squarain family,
Fluorescent substance of chelated lanthanide family,
Fluorescent substance of BODIPY (registered trademark) family (produced by Invitrogen),
Fluorescent substance of naphthalenesulfonic acid family,
Fluorescent substance of pyrene family,
Fluorescent substance of triphenylmethane family, and
Alexa Fluor (registered trademark) dye series (produced by Invitrogen).

Examples of a surfactant contained in an antibody dispersion for preservation include Tween 20 (polyoxyethylene sorbitan monolaurate), Triton X-100, and Span 80 (sorbitan monooleate). The amount of a surfactant contained in an antibody dispersion for preservation is preferably from 0.00001 to 1 mass% of a surfactant in 100 mass% of an antibody dispersion for

The amount of a monovalent metal salt contained in an antibody dispersion for preservation is usually from 10 to 300 mM, and preferably from 30 to 250 mM

The amount of a divalent metal salt contained in an antibody dispersion for preservation is usually from 0 to 0.8 mM, and preferably from 0 to 0.6 mM

Meanwhile, the sum of the concentration of a monovalent metal salt, and the concentration of a divalent metal salt contained in the antibody dispersion for measurement is preferably higher than the sum of the concentration of a monovalent metal salt and the concentration of a divalent metal salt contained in an antibody dispersion for preservation by 0.5 to 250 mM, because aggregation of the antibody even at the time of the antigen-antibody reaction may be suppressed so that the emission intensity of a fluorescent substance may be prohibited from decreasing.

Examples of a monovalent metal salt and a divalent metal salt contained in an antibody dispersion for preservation include those exemplified in the above paragraph for an antibody dispersion for measurement.

<Solution containing metal salt>

A solution containing a metal salt used according to the present invention is a solution containing a divalent metal salt, and may be a solution containing a divalent metal salt and a monovalent metal salt. An antibody dispersion for measurement according to the present invention may be obtained by adding a solution containing a metal salt to an antibody dispersion for preservation.

The solution containing a metal salt preferably contains a solvent such as water, a surfactant, *etc.* in addition to the metal salt. Further, it is preferable that the solution containing a metal salt is a buffer solution from the viewpoint of stabilization of the pH. When it is a buffer solution, examples thereof may include an acetate buffer, a phosphate buffer, a Tris buffer, a HEPES buffer, a citrate buffer, a citrate-phosphate buffer, and a borate buffer. As the buffer solution, among a phosphate buffer, a Tris buffer, and a HEPES buffer, a phosphate buffered saline (PBS), a Tris buffered saline (TBS), and a HEPES buffered saline, which are almost isotonic with a body fluid, are preferable.

Examples of a divalent metal salt and a monovalent metal salt contained in the solution containing a metal salt include the divalent metal salt and the monovalent metal salt exemplified in the above paragraph for an antibody dispersion for measurement.

The concentration of a divalent metal salt contained in the solution containing a metal salt is preferably from 1.0 to 80 mM, and more preferably from 1.0 to 65 mM

In a case where a polymer compound such as a protein is contained as an antibody in an antibody dispersion for preservation, when a specific amount of a divalent metal salt is added to the antibody dispersion for preservation the solubility of the polymer compound is enhanced, and the inventors conjecture that a phenomenon called "salting-in" occurs, and conceive further that there appears an influence of the Hofmeister series (The Hofmeister series refers to the ion order of the precipitation inducing ability). Especially, the inventors conjecture that a certain concentration of a divalent metal salt affects hydrated water present on the surface of a polymer compound such as a protein to keep a proper intermolecular distance contributing to dissolution stability.

The monovalent metal salt contained in the solution containing a metal salt is added, when an antibody dispersion for measurement is prepared by mixing an antibody dispersion for preservation and a solution containing a metal salt, in order not to dilute excessively the concentration of a monovalent metal salt, or in order to adjust the concentration of a monovalent metal salt. There is no particular restriction on the concentration of a monovalent metal salt in the solution containing a metal salt, and it may be appropriately adjusted according to the concentration of the monovalent metal salt in an antibody dispersion for preservation, or adjusted to make the concentration of the monovalent metal salt in an antibody dispersion for measurement within a specific range, more particularly it is usually from 100 to 600 mM, and preferably from 150 to 500 mM

Examples of a surfactant contained in the solution containing a metal salt may include the same surfactants as contained in the above antibody dispersion for preservation. The surfactant contained in an antibody dispersion for preservation and the surfactant contained in the solution containing a metal salt may be the same or different.

«Light emission ratio of fluorescence-labeled antibody»

When an antibody dispersion for measurement according to the present invention contains a fluorescence-labeled antibody, it is preferable that the same exhibits an excellent luminous efficiency.

Specifically, the emission intensity of the antibody dispersion for measurement is preferably 60 to 99%, when the concentration of the fluorescence-labeled antibody contained in the antibody dispersion for measurement is X [mol/L] and the emission intensity of a dispersion of a bare fluorescent substance to constitute a fluorescence-labeled antibody (at a concentration of X [mol/L]) is regarded as 100%. In this regard, the concentration of the fluorescence-labeled antibody means herein the concentration of a fluorescent substance to constitute the fluorescence-labeled antibody.

When an antibody dispersion for measurement, and a dispersion containing the bare fluorescent substance to constitute the fluorescence-labeled antibody in the antibody dispersion for measurement at the same concentration are prepared and both the emission intensities are compared, the emission intensity of the antibody dispersion for measurement should preferably be within the above range with respect to the emission intensity of the dispersion containing the bare fluorescent substance as 100%.

The emission intensity of an antibody dispersion for measurement may be calculated according to the following formula, and is more preferably from 70 to 99%, and further preferably from 80 to 99%.

$$(\text{Emission intensity of fluorescence-labeled antibody}) / (\text{Emission intensity of bare fluorescent substance})$$

$$\times\ 100$$

There is no particular restriction on a method of measuring the emission intensity, and it may be measured using a general fluorophotometer under an appropriate measurement condition.

<<Production method of antibody dispersion for measurement>>

A production method according to the present invention for an antibody dispersion for measurement comprising a monovalent metal salt and a divalent metal salt, wherein the concentration of the monovalent metal salt is from 50 to 500 mM and the concentration of the divalent metal salt is from 1.0 to 50 mM, comprises a step of adding a divalent metal salt to an antibody dispersion for preservation containing a monovalent metal salt at a concentration of from 10 to 300 mM

It is preferable that an antibody dispersion for preservation contains a fluorescence-labeled antibody. The details of an antibody dispersion for preservation and an antibody dispersion for measurement are as described in the above paragraphs on the antibody dispersion for preservation and the antibody dispersion for measurement.

The step of adding a divalent metal salt is preferably a step of adding a divalent metal salt and a monovalent metal salt, and a step of adding a monovalent metal salt may be added after the step of adding the divalent metal salt.

The step of adding a divalent metal salt in the production method according to the present invention may be carried out by adding directly a metal salt to an antibody dispersion for preservation, or by adding a solution containing a metal salt.

Meanwhile, the step of adding a monovalent metal salt may be carried out by adding directly a metal salt to an antibody dispersion for preservation having undergone the step of adding a divalent metal salt, or by adding a solution containing a metal salt. Examples of a solution containing a metal salt used in the step of adding a monovalent metal salt include a solution obtained by removing a divalent metal salt from the solution containing a metal salt described in the paragraph of the solution containing a metal salt.

«Kit for preparing antibody dispersion for measurement»

A kit for preparing an antibody dispersion for measurement according to the present invention comprises an antibody dispersion for preservation containing a monovalent metal salt at a concentration of from 10 to 300 mM, and a solution containing a divalent metal salt, wherein an antibody contained in the antibody dispersion for preservation is a fluorescence-labeled antibody.

«Method for measuring biological substance»

A method for measuring a biological substance according to the present invention needs to include the following steps (A) to (D) in order to quantify a target biological substance:

(A) a step for obtaining an antibody dispersion for measurement, in which a monovalent metal salt and a divalent metal salt are contained, and the concentration of the monovalent metal salt is from 50 to 500 mM and the concentration of the divalent metal salt is from 1.0 to 50 mM, by adding a divalent metal salt to an antibody dispersion for preservation, in which a fluorescence-labeled antibody and a monovalent metal salt are contained, and the concentration of the monovalent metal salt is from 10 to 300 mM,
(B) a step for supplying a specimen containing a target biological substance onto a sensor chip,
(C) a step for fluorescently labeling the specimen containing a target biological substance by supplying the antibody dispersion for measurement obtained in the step (A) onto the sensor chip having undergone the step (B), and
(D) a step for detecting the fluorescence-labeled specimen containing a target biological substance by performing a fluorescence assay.

In the quantification by a method for measuring a biological substance according to the present invention, for example, a standard sample of a measurement target compound is added to a commercially available biological sample (such as blood), and the above steps are carried out to prepare a calibration curve. The concentration of measurement target compound in a measured biological sample may be known through fitting the signal intensity obtained from a measured biological sample with the calibration curve.

When an antibody is stored for a long time in a state where a divalent metal salt has been added, antibody aggregation or the like may occur, and the condition of the antibody may be changed. Therefore, it is preferable that the step (C) is performed within 60 min, preferably within 10 min, after implementation of the step (A).

A method for measuring a biological substance according to the present invention may be applied in carrying out a fluorescence assay such as surface plasmon field-enhanced fluorescence spectroscopy (SPFS).

«Specimen»

A specimen which is analyzed using an antibody dispersion for measurement according to the present invention will be described in detail below.

As the specimen, a specimen that may contain a detection target substance and a contaminant is used. The specimen that may contain a detection target substance and a contaminant may be a specimen which actually contains them, or a specimen which actually does not contain them. For example, it may be a specimen derived from a patient of a specific disease having a high possibility of containing the detection target substance, or a specimen derived from a healthy person having a low possibility of containing the detection target substance. Although an object, from which a specimen is collected, is typically a human, however it may also be a model animal of a human disease, namely a non-human mammal such as a mouse, a rat, a guinea pig, a rabbit, a goat, a cat, a dog, a pig, and a monkey.

Examples of a specimen may include a biological sample and a sample of biological origin, such as blood, urine, cerebrospinal fluid, saliva, cell, tissue, and organ as well as preparations thereof (for example, biopsy specimen). For example, blood or urine is a specimen, which may contain a glycoprotein usable as a diagnostic marker, preferable as a specimen used according to the present invention.

A liquid specimen, such as blood, serum, plasma, urine, cerebrospinal fluid, and saliva, may be used as it is as a specimen, or diluted with an appropriate specimen diluent solution and then used as a specimen. A solid specimen, such as cell, tissue, and organ, may be homogenized with an appropriate buffer solution in an amount of about 2 to 10 times the volume of the solid specimen, and the suspension or its supernatant may be used as it is, or after further dilution with a specimen diluent solution as a specimen.

In a preferred example of the embodiment, blood is used as specimen. In this case, the blood may be whole blood, or may be serum or plasma prepared from whole blood. For example, for the sake of quick measurement, whole blood may be used as the specimen, or for the purpose of accurate quantification, blood cell components are removed from the whole blood by centrifugation, *etc.* to prepare serum or plasma, which may be used as the specimen. At the time of blood collection, an anticoagulant is usually added to whole blood, and when a measurement utilizing surface plasmon field-enhanced fluorescence spectroscopy (SPFS) described later is scheduled, the whole blood, serum, or plasma is diluted with a specimen diluent solution in order to adjust the concentration to an appropriate level, and another reagent, *etc.,* which may be necessary, is further added. If necessary, such an anticoagulant, or another reagent, *etc.* may be added according to the present invention.

<Specimen diluent solution>

As a main component for a specimen diluent solution, for example, phosphate buffered saline (PBS), Tris buffered saline (TBS), or HEPES buffered saline (HBS) may be used.

A specimen diluent solution according to the present invention preferably contains, for example, carboxymethyldextran (CMD). This is because nonspecific adsorption of contaminants in a specimen to a ligand or the like may be suppressed so that the quantification accuracy on an antigen may be improved.

A specimen diluent solution according to the present invention desirably contains a surfactant. When it contains a surfactant, for example, in a case where the contaminant is a lipid, the surfactant acts thereon to form micelles, which suppress aggregation of the contaminant in the specimen.

In this case, there is no particular restriction on a surfactant, however an anionic surfactant is preferable from the viewpoint of utilization of a Coulomb repulsive force by realizing the same charged state as CMD. For example, sodium dodecyl sulfate (SDS), a monoalkyl sulfate, an alkyl polyoxyethylene sulfate, an alkylbenzene sulfonate, and a monoalkyl phosphate may be used.

In this case, a surfactant is desirably contained in an amount of from 0.00001 to 1 mass% in a specimen diluent solution as 100 mass%. Further, the concentration of a surfactant in a specimen after dilution is preferably from 0.000005 to 0.5 mass%. When a surfactant, such as Tween 20, or Triton X-100, is used, it is preferable to contain the surfactant at 0.00001 to 1 mass% in a specimen diluent solution as 100 mass%.

Examples

Next, the present invention will be described in more detail by way of Examples, provided that the present invention be not limited in any way by the Examples.

«Production of sensor chip»

A glass dielectric unit ("S-LAL10" produced by Ohara Inc.) having a refractive index (nd) of 1.72 and a thickness of 1 mm was subjected to plasma cleaning, and a chromium thin film was formed on one side of the support by sputtering.

Thereafter, a gold thin film was further formed on its surface. The thickness of the chromium thin film was from 1 to 3 nm, and the thickness of the gold thin film was from 42 to 47 nm. The substrate thus obtained was immersed in 10 mL of an ethanol solution of 10-amino-1-decanethiol adjusted to 1 mM for 24 hours to form a SAM (Self-Assembled Monolayer) on one side of the gold thin film. The substrate was taken out from the ethanol solution, washed with ethanol and isopropanol respectively, and then dried using an air gun.

Subsequently, the support on which the SAM was formed was immersed in a 2-(morpholine)ethanesulfonic acid buffered saline (MES) (ionic strength: 10 mM) with a pH of 7.4 containing 1 mg/mL of carboxymethyldextran (CMD) having a molecular weight of 500,000, 0.5 mM of $N$-hydroxysuccinimide (NHS), and 1 mM of a water-soluble carbodiimide (WSC) for 1 hour to immobilize CMD on the SAM. The product was immersed and immersed in a 1 N NaOH aqueous solution for 30 min to hydrolyze the unreacted succinate.

Next, the product was immersed in MES containing 50 mM of NHS and 100 mM of WSC for 1 hour, and then immersed in a solution of anti-cTnI IgGl monoclonal antibody (MF4; 2.5 $\mu$g/mL, produced by HyTest Ltd.) for 30 min to immobilize the primary antibody on the CMD.

<<Preparation of specimen>>

A specimen was prepared by adding a specimen diluent solution to cardiac troponin I (cTnI) which was a standard antigen.

A specimen was obtained by mixing cTnI and a specimen diluent solution such that the concentration of cTnI after dilution with the specimen diluent solution became 100 pg/mL.

For the specimen diluent solution, 10 mL of 10 × Tris buffered saline (TBS) (pH 7.4) (produced by Nippon Gene Co., Ltd.), 0.1 mg of sodium carboxymethyldextran (weight-average molecular weight of 10000, produced by Tokyo Chemical Industry Co., Ltd.), and a surfactant (Tween 20) were mixed. The surfactant was used in an amount equivalent to 0.05 mass% of the specimen diluent solution.

«Preparation of fluorescence-labeled antibody dispersion»

A fluorescence-labeled antibody dispersion was prepared.

Using a solution of an anti-cTnI IgG2a monoclonal antibody (4C2; 2.5 mg/mL, produced by HyTest Ltd.), and a labeling kit of Alexa Fluor (registered trademark) 647 (produced by Molecular Probes), the two were reacted by mixing them with stirring at room temperature (25C) for 60 min according to the procedures set forth in the instruction for use of the kit. Thereafter, through purification using a molecular weight cutoff filter (produced by Nihon Millipore K.K.) an Alexa Fluor (registered trademark) 647-labeled anti-cTnI IgG2a monoclonal antibody was obtained.

[Examples 1 to 7, and Comparative Example 1]

«Preparation of antibody dispersion for preservation»

The antibody concentration was determined by measuring the absorbance of the fluorescence-labeled antibody dispersion prepared as above. Based on the determined antibody concentration, a solution was adjusted with the specimen diluent solution such that the final antibody concentration became 1 mg/mL.

For Examples 1 to 7 and Comparative Example 1, to 200 $\mu$L of the above 1 mg/mL fluorescence-labeled antibody dispersion, 1 mL of a 10× phosphate buffered saline (PBS) (10 times concentration PBS stock solution, produced by Nippon Gene Co., Ltd.), 800 mg of albumin (derived from bovine serum, IgG/protease-free, produced by Wako Pure Chemical Industries, Ltd.), and 5 mg of a surfactant (Tween 20), ultrapure water was added to reach 10 mL, thereby obtaining an antibody dispersion for preservation.

The concentrations of metal salts in an antibody dispersion for preservation are shown in Table 1. In this regard, the concentrations with respect to NaCl and KCl are derived from PBS.

[Table 1]

| Table 1 Concentration of metallic salts in antibody dispersion for preservation | | |
|---|---|---|
| NaCl(mM) | KCl(mM) | $MgCl_2$(mM) |
| 157 | 5 | 0 |

«Preparation of cleaning solution»

A cleaning solution was obtained by dissolving a surfactant (Tween 20) in a phosphate buffered saline (PBS) at 0.05 mass%

<<Preparation of antibody dispersion for measurement>>

An antibody dispersion for measurement was obtained by mixing 50 $\mu$L of the above an antibody dispersion for preservation and 50 $\mu$L of the following solution containing a metal salt. In this regard, the antibody dispersion for measurement was prepared 5 min before its addition to a sensor chip.
A solution containing a metal salt was prepared and used by adding ultrapure water to sodium chloride (NaCl), potassium chloride (KCl), and magnesium chloride ($MgCl_2$) in a measuring cylinder to reach 10 mL, such that the concentrations set forth in Table 2 with respect to each of Examples and Comparative Example were satisfied.
The concentrations of the metal salts contained in the solutions are shown in Table 2.

[Table 2]

| Table 2 Concentrations of metal salts in solution containing metal salts | | | |
|---|---|---|---|
| | NaCl(mM) | KCl(mM) | $MgCl_2$(mM) |
| Example 1 | 157 | 5 | 2 |
| Example 2 | 157 | 5 | 20 |
| Example 3 | 157 | 5 | 30 |
| Example 4 | 157 | 5 | 60 |
| Example 5 | 217 | 5 | 60 |
| Example 6 | 357 | 5 | 60 |
| Example 7 | 437 | 5 | 60 |
| Comparative Example 1 | 157 | 5 | 0 |

The concentrations of metal salts in the obtained antibody dispersions for measurement are shown in Table 3.

[Table 3]

| Table 3 Concentrations of metal salts in antibody dispersion for measurement | | | |
|---|---|---|---|
| | NaCl(mM) | KCl(mM) | $MgCl_2$(mM) |
| Example 1 | 157 | 5 | 1 |
| Example 2 | 157 | 5 | 10 |
| Example 3 | 157 | 5 | 15 |
| Example 4 | 157 | 5 | 30 |
| Example 5 | 187 | 5 | 30 |
| Example 6 | 257 | 5 | 30 |
| Example 7 | 297 | 5 | 30 |
| Comparative Example 1 | 157 | 5 | 0 |

«Quantitative determination of antigen»

Each antibody dispersion for measurement obtained as above was sent to a sensor chip, and a fluorescence assay was performed.

<Fluorescence measurement>

Through a flow channel of the sensor chip, 0.1 mL of the sample solution (specimen) was circulated for 25 min, and then a Tris-buffered saline (TBS) containing 0.05 mass% of Tween 20 was circulated for washing for 10 min.

Next, 0.1 mL of an antibody dispersion for measurement was circulated for 5 min, and then the TBS containing 0.05 mass% of Tween 20 was circulated for 10 min for washing. Thereafter, a plasmon excitation sensor was irradiated with a laser beam (640 nm, 40 $\mu$W) from a surface, on which a metal thin film was not formed, of a dielectric member made of glass through a prism (manufactured by Sigmakoki Co., Ltd.), and the amount of fluorescence emitted form an excited fluorescent substance was measured in terms of the amount of light (signal value) detected with a photomultiplier tube (PMT), which was regarded as the signal value (S) of the sample solution containing an antigen.

A sample solution not containing cardiac troponin I (cTnI) which was the standard antigen was measured similarly to measure the background value (B).

<Evaluation on quantitativity of antigen>

From the signal value (S) and the background value (B) obtained above, the S/B ratio was calculated. Based on the S/B value of Comparative Example 1, the quantitativities of antigen in Examples 1 to 7 were evaluated. The results are shown in the following Table 4.

[Table 4]

| Table 4 Evaluation on quantitativity of antigen | |
|---|---|
| Example 1 | 1.1 |
| Example 2 | 1.4 |
| Example 3 | 1.8 |
| Example 4 | 1.5 |
| Example 5 | 1.6 |
| Example 6 | 1.8 |
| Example 7 | 1.6 |
| Comparative Example 1 | 1 |

«Measurement of light emission ratio of fluorescence-labeled antibody»

The emission intensity of the antibody dispersion for measurement obtained as above was measured with a fluorometer (NanoDrop 3000, produced by Thermo Fisher Scientific Inc.). Similarly, the emission intensity of a dispersion of the fluorescent substance Alexa Fluor (registered trademark) 647 (produced by Molecular Probes) prepared by the following method was measured. Then, the light emission ratio of a fluorescence-labeled antibody was calculated according to Formula (I):

$$\text{(Emission intensity of antibody dispersion for measurement)} / \text{(Emission intensity of fluorescent substance)} \times 100 \quad \text{Formula (I)}$$

The molecular weight (Mw) of the anti-cTnI IgG2a monoclonal antibody is about 15000, the molecular weight (Mw) of Alexa Fluor (registered trademark) 647 is about 1200, and an Alexa Fluor (R) 647 -labeled anti-cTnI IgG2a monoclonal antibody has two Alexa Fluor (registered trademark) 647 per anti-cTnI IgG2a monoclonal antibody. Therefore, the molecular weight (Mw) of the Alexa Fluor (R) 647-labeled anti-cTnI IgG2a monoclonal antibody is about 17,400. Since the amount of a fluorescence-labeled antibody in an antibody dispersion for preservation is 0.02 mg/mL, and the amount of a fluorescence-labeled antibody in an antibody dispersion for measurement is 0.01 mg/mL, the concentration of Alexa Fluor (registered trademark) 647 contained in an antibody dispersion for measurement is 1.149 µM (µmol/L).

The dispersion of Alexa Fluor (registered trademark) 647 (produced by Molecular Probes) was prepared by mixing the Alexa Fluor (registered trademark) 647, a 10 × phosphate buffered saline (PBS) (10 times concentration PBS stock solution, produced by Nippon Gene Co., Ltd.), and ultrapure water, such that the concentration of Alexa Fluor (registered trademark) 647 became 1.149 µM (µmol/L), the concentration of NaCl became 157 mM, the concentration of KCl became 5 mM, and the concentration of $MgCl_2$ became 0 mM

The light emission ratio of the fluorescence-labeled antibody is shown in Table 5.

[Table 5]

| Table 5 Light emission ratio (%) of fluorescence-labeled antibody | |
|---|---|
| Example 1 | 60 |
| Example 2 | 76 |
| Example 3 | 98 |
| Example 4 | 82 |
| Example 5 | 87 |
| Example 6 | 98 |
| Example 7 | 87 |
| Comparative Example 1 | 54 |

**Claims**

1. An antibody dispersion for measurement comprising a monovalent metal salt and a divalent metal salt, wherein the concentration of the monovalent metal salt is from 50 to 500 mM and the concentration of the divalent metal salt is from 1.0 to 50 mM

2. The antibody dispersion for measurement according to claim 1 comprising a fluorescence-labeled antibody.

3. The antibody dispersion for measurement according to claim 1 or 2 obtained by adding a divalent metal salt to an antibody dispersion for preservation containing a monovalent metal salt at a concentration of from 10 to 300 mM

4. The antibody dispersion for measurement according to claim 1 or 2 obtained by adding a divalent metal salt and a monovalent metal salt to an antibody dispersion for preservation containing a monovalent metal salt at a concentration of from 10 to 300 mM

5. The antibody dispersion for measurement according to claim 3 or 4, wherein the sum of the concentration of the monovalent metal salt and the concentration of the divalent metal salt contained in the antibody dispersion for measurement is higher than the sum of the concentration of the monovalent metal salt and the concentration of the divalent metal salt contained in the antibody dispersion for preservation by from 0.5 to 250 mM

6. The antibody dispersion for measurement according to any one of claims 1 to 5 containing a fluorescence-labeled antibody, wherein the emission intensity of the antibody dispersion for measurement is from 60 to 99%, when the concentration of the fluorescence-labeled antibody contained in the antibody dispersion for measurement is X [mol/L] and the emission intensity of a dispersion of a bare fluorescent substance to constitute a fluorescence-labeled antibody (at a concentration of X [mol/L]) is regarded as 100%.

7. The antibody dispersion for measurement according to any one of claims 1 to 6, wherein the divalent metal salt is at least one metal salt selected from a magnesium salt and a calcium salt.

8. The antibody dispersion for measurement according to any one of claims 1 to 7, wherein the monovalent metal salt is at least one metal salt selected from a sodium salt, a potassium salt, and a lithium salt.

9. A production method of an antibody dispersion for measurement comprising a monovalent metal salt and a divalent metal salt, wherein the concentration of the monovalent metal salt is from 50 to 500 mM and the concentration of the divalent metal salt is from 1.0 to 50 mM, and

wherein the method comprises a step of adding a divalent metal salt to an antibody dispersion for preservation containing a monovalent metal salt at a concentration of from 10 to 300 mM

10. The production method of an antibody dispersion for measurement according to claim 9, wherein the antibody dispersion for preservation contains a fluorescence-labeled antibody.

11. The production method of an antibody dispersion for measurement according to claim 9 or 10, wherein the step of adding a divalent metal salt is a step of adding a divalent metal salt and a monovalent metal salt.

12. The production method of an antibody dispersion for measurement according to any one of claims 9 to 11 comprising a step of adding a monovalent metal salt after the step of adding a divalent metal salt.

13. The production method of an antibody dispersion for measurement according to any one of claims 9 to 12, wherein the divalent metal salt is at least one metal salt selected from a magnesium salt and a calcium salt.

14. The production method of an antibody dispersion for measurement according to any one of claims 9 to 13, wherein the monovalent metal salt is at least one metal salt selected from a sodium salt, a potassium salt, and a lithium salt.

15. A kit for preparing an antibody dispersion for measurement comprising an antibody dispersion for preservation containing a monovalent metal salt at a concentration of from 10 to 300 mM, and a solution containing a divalent metal salt,

wherein an antibody contained in the antibody dispersion for preservation is a fluorescence-labeled antibody.

16. A measuring method of a biological substance for quantifying a target biological substance comprising the following steps (A) to (D):

(A) a step for obtaining an antibody dispersion for measurement, in which a monovalent metal salt and a divalent metal salt are contained, and the concentration of the monovalent metal salt is from 50 to 500 mM and the concentration of the divalent metal salt is from 1.0 to 50 mM, by adding a divalent metal salt to an antibody dispersion for preservation, in which a fluorescence-labeled antibody and a monovalent metal salt are contained, and the concentration of the monovalent metal salt is from 10 to 300 mM,
(B) a step for supplying a specimen containing a target biological substance onto a sensor chip,
(C) a step for fluorescently labeling the specimen containing a target biological substance by supplying the antibody dispersion for measurement obtained in the step (A) onto the sensor chip having undergone the step (B), and
(D) a step for detecting the fluorescence-labeled specimen containing a target biological substance by performing a fluorescence assay.

17. The measuring method of a biological substance according to claim 16, wherein the fluorescence assay is carried out by surface plasmon field-enhanced fluorescence spectroscopy (SPFS).

Europäisches
Patentamt

European
Patent Office

Office européen
des brevets

## EUROPEAN SEARCH REPORT

Application Number

EP 18 19 5594

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 141 343 A2 (BOEHRINGER MANNHEIM GMBH [DE]) 15 May 1985 (1985-05-15) | 1-15 | INV. G01N33/543 |
| A | * p. 4, last paragraph - p. 6, first paragraph, pp. 13-16, Ex. 2-4 * | 16,17 | C07K16/18 G01N21/64 |
| | ----- | | |
| X | US 6 242 265 B1 (GIESENDORF BERNHARD [DE]) 5 June 2001 (2001-06-05) * col. 1, line 46 - col. 4, last line * | 1-17 | |
| | ----- | | |
| X | EP 0 710 838 A1 (WAKO PURE CHEM IND LTD [JP]) 8 May 1996 (1996-05-08) * p. 2, lines 29-41, pp. 4-8, Ex 1-4, claims 1-5 * | 1-17 | |
| | ----- | | |
| X | EP 2 930 514 A1 (KONICA MINOLTA INC [JP]) 14 October 2015 (2015-10-14) * p. 3, [0016] - p. 10, [0088]p. 12, [0103] - p. 15, [0116], Table 1 * | 1-17 | |
| | ----- | | |

TECHNICAL FIELDS
SEARCHED      (IPC)

G01N
C07K

The present search report has been drawn up for all claims

| Place of search | Date of completion of the search | Examiner |
|---|---|---|
| Munich | 27 November 2018 | R. von Eggelkraut-G. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 18 19 5594

This annex lists the patent family members relating to the patent documents cited in the above-mentioned European search report.
The members are as contained in the European Patent Office EDP file on
The European Patent Office is in no way liable for these particulars which are merely given for the purpose of information.

27-11-2018

| Patent document cited in search report | | | Publication date | Patent family member(s) | | | Publication date |
|---|---|---|---|---|---|---|---|
| EP 0141343 | A2 | | 15-05-1985 | DE | 3338836 | A1 | 09-05-1985 |
| | | | | EP | 0141343 | A2 | 15-05-1985 |
| | | | | JP | H0235260 | B2 | 09-08-1990 |
| | | | | JP | S60173470 | A | 06-09-1985 |
| | | | | US | 4746605 | A | 24-05-1988 |
| US 6242265 | B1 | | 05-06-2001 | AT | 125948 | T | 15-08-1995 |
| | | | | AU | 630410 | B2 | 29-10-1992 |
| | | | | CA | 2008060 | A1 | 19-07-1990 |
| | | | | DE | 3901458 | A1 | 26-07-1990 |
| | | | | EP | 0379133 | A2 | 25-07-1990 |
| | | | | ES | 2076232 | T3 | 01-11-1995 |
| | | | | JP | 3018364 | B2 | 13-03-2000 |
| | | | | JP | H02228560 | A | 11-09-1990 |
| | | | | US | 6242265 | B1 | 05-06-2001 |
| EP 0710838 | A1 | | 08-05-1996 | EP | 0710838 | A1 | 08-05-1996 |
| | | | | TW | 296432 | B | 21-01-1997 |
| | | | | US | 5962340 | A | 05-10-1999 |
| EP 2930514 | A1 | | 14-10-2015 | EP | 2930514 | A1 | 14-10-2015 |
| | | | | JP | 2017198706 | A | 02-11-2017 |
| | | | | JP | WO2014087802 | A1 | 05-01-2017 |
| | | | | US | 2015309019 | A1 | 29-10-2015 |
| | | | | WO | 2014087802 | A1 | 12-06-2014 |

EPO FORM P0459

For more details about this annex : see Official Journal of the European Patent Office, No. 12/82

## REFERENCES CITED IN THE DESCRIPTION

**Patent documents cited in the description**

• JP H448266 B